# EUROPEAN PATENT SPECIFICATION

(11) **EP 1 605 983 B1**
(45) Date of publication and mention of the grant of the patent: **05.01.2011**
(21) Application number: 04723565.0
(22) Date of filing: 26.03.2004
(51) Int. Cl.: A61C 8/00, A61K 6/087, A61L 27/34, C08L 85/02, C08G 79/02

(54) **COATED DENTAL IMPLANTS**
BESCHICHTETE DENTALIMPLANTATE
IMPLANTS DENTAIRES TRAITES

(30) Priority: 26.03.2003 US 457694 P
(43) Date of publication of application: 21.12.2005
(73) Proprietor: CeloNova BioSciences Germany GmbH, 89077 Ulm (DE)
(72) Inventor: DENK, Roman, 89197 Weidenstetten (DE); WAGNER, Ulrich, 76227 Karlsruhe (DE)
(74) Representative: Maughan, Sophie Louise
(86) International application number: PCT/EP2004/003262
(87) International publication number: WO 2004/084966

(56) References cited:
- EP-A- 1 312 635
- EP-A- 1 344 538
- WO-A-02/13882
- WO-A-03/015719
- WO-A-2004/004795
- DE-A- 10 019 982
- DE-A- 19 613 048
- US-A- 4 592 755
- LEMMOUCHI Y ET AL: "BIODEGRADABLE POLYPHOSPHAZENES FOR DRUG DELIVERY" MACROMOLECULAR SYMPOSIA, WILEY VCH, WEINHEIM, DE, vol. 123, 1 September 1997 (1997-09-01), pages 103-112, XP000727295 ISSN: 1022-1360

## Description

### FIELD OF THE INVENTION

The present invention relates to coated dental implants which allow a sufficient take of the gingiva onto said dental implants, thereby preventing *inter alia* the deepening of the gingival sulcus and bacterial infections of the gingiva and methods of using said coated dental implants for preventing the deepening of the gingival sulcus and the formation of bacterial infection upon their implantation into a patient.

### BACKGROUND OF THE INVENTION

One of the most serious complications known from artificial implants is an increased deposition of thrombocytes at the surface of implants in a patient. A possibility to deal with this complication is to use coated implants. For example, DE 196 13 048 describes artificial implants having a biocompatible coating which contains a compound with antithrombogenic properties.

A frequent problem of artificial dental implants lies in an insufficient connection between the gingiva and the dental implant; i.e. an insufficient tissue integration of the dental implant onto the gingiva upon implantation into a patient. An insufficient connection often results in the deepening of the gingival sulcus and an increased risk of bacterial infections. This can lead, in the worst case, to severe inflammation in connection with a loss of the dental implant.

EP 1312635 (POLYZENIX GMBH) is state of the art under Article 54(3) EPC, which describes polyphosphazene derivatives and their use, having excellent biocompatible properties and imparting bacterial resistance to a coating of an article such as a medical device. In particular, the coating is applied on at least part of a surface of e.g. said medical device and can be sued for preventing and/pr reducing an inflammatory response upon application of said medical device to a patient.

WP 03/015719 (ROMAN et al) describes a device, comprising a substrate essentially based on nitinol with at least a partial layer, or coating based on at least one polyphosphazene derivative.

At present, no promising solution for a sufficient take of the gingiva onto dental implants without or at least reduced deepening of gingival sulcus and/or without or at least reduced risk of bacterial infections are known in the art.

### SUMMARY OF THE INVENTION

According to an aspect of the present invention there is provided a dental implant as claimed in Claim 1. According to a further aspect of the present invention there is provided a method for manufacturing a dental implant as claimed in Claim 13.

It is an object of the present invention to provide an artificial dental implant which allows a sufficient take of the gingiva onto the dental implant, thereby preventing substantially the deepening of the gingival sulcus, at least minimizing the risk of bacterial infections and improving the long-term tolerance of the dental implant, upon its implantation into a patient.

In particular, the present invention relates to a dental implant comprising a dental implant material comprising titanium having a biocompatible coating which is applied to the surface of the dental implant material that contacts the gingival of a patient upon implantation of the dental implant, wherein the biocompatible coating allows a sufficient take of the gingiva onto the dental implant resulting in (i) substantially no deepening of the gingival sulcus, (ii) substantially no bacterial infections of the gingiva close to the coated dental implant and (iii) an improved long-term tolerance of the coated dental implant.

The expression "at least part of the surface" means that part of the surface of the implant material, which should come into contact with the gingiva of the patient upon implantation.

It is another object of the present invention to provide methods of (i) preventing deepening of the gingival sulcus at a dental implant, (ii) preventing bacterial infections of the gingiva at a dental implant and (iii) taking the gingiva onto a dental implant, upon implantation of the dental implant of the present application into a patient.

### DETAILED DESCRIPTION OF THE INVENTION

According to an embodiment of the present invention, the dental implant comprises an implant material comprising titanium having a biocompatible coating which is applied to the surface of the dental implant material that contacts the gingival of a patient upon implantation of the dental implant, wherein said biocompatible coating contains a polymer having the general formula (I) 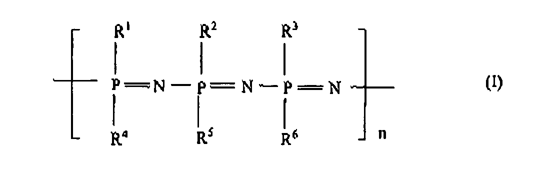 wherein
n is from 2 to ∞
R¹ to R⁶ are the same or different and represent an alkoxy, alkylsulfonyl, dialkylamino or aryloxy group, or a heterocycloalkyl or heteroaryl group having nitrogen as the heteroatom.

In the polymer of formula (I) it is preferred that at least one of the groups R¹ and R² is an alkoxy group substituted with at least one fluorine atom.

In the polymer of formula (1), the alkyl groups in the alkoxy, alkylsulfonyl and dialkylamino groups are, for example, straight-chain or branched-chain alkyl groups having 1 to 20 carbon atoms, wherein the alkyl groups can be substituted, for example, with at least one halogen atom, such as a fluorine atom.

Examples of alkoxy groups are methoxy, ethoxy, propoxy and butoxy groups, which preferably can be substituted with at least one fluorine atom. The 2,2,2-trifluoroethoxy group is particularly preferred.

Examples of alkylsulfonyl groups are methylsulfonyl, ethylsulfonyl, propylsulfonyl and butylsulfonyl groups.

Examples of dialkylamino groups are dimethylamino, diethylamino, dipropylamino and dibutylamino groups.

The aryl group in the aryloxy group is, for instance, a compound having one or more aromatic ring systems, wherein the aryl group can be substituted, for instance, with at least one alkyl group as defined above.

Examples of aryloxy groups are phenoxy and naphthoxy groups, and derivatives thereof.

The heterocycloalkyl group is, for example, a ring system containing 3 to 7 atoms, at least one of the ring atoms being a nitrogen atom. The heterocycloalkyl group can, for example, be substituted with at least one alkyl group as defined above. Examples of heterocycloalkyl groups are piperidinyl, piperazinyl, pyrrolidinyl and morpholinyl groups, and derivatives thereof.

The heteroaryl group is, for example, a compound with one or more aromatic ring systems, wherein at least one ring atom is a nitrogen atom. The heteroaryl group can, for example, be substituted with at least one alkyl group as defined above. Examples of heteroaryl groups are pyrrolyl, pyridinyl, pyridinolyl, isoquinolinyl and quinolinyl groups, and derivatives thereof.

In a preferred embodiment of the present invention, the biocompatible coating contains the polymer bis-poly-trifluorethoxy-polyphosphazene.

The production of polymers of formula (I), such as bis-poly-trifluorethoxy-polyphosphazene, starting with hexachlorocyclotriphosphazene, is known in the art. The polymerization of hexachlorocyclotriphosphazene is expensively described in Korsak et al., Acta Polymerica 30, No. 5, pages 245-248 (1979). Esterification of the polydichlorophosphazene produced by the polymerization is described in Fear, Thower and Veitch, J. Chem. Soc., page 1324 (1958).

The biocompatible coating of the dental implant according to the present invention has, for example, a thickness from about 1 nm to about 100 µm, preferably from about 10 nm to about 10 µm, and more preferably up to about 1 µm.

Also described herein are other suitable materials which can be used for the uncoated dental implant. It can be any implant material useful for dental implants. In particular, the dental implant material can be a metal, an alloy, a polymeric material or a ceramic material. For example, the metallic material can be titanium as in the present invention. In a preferred embodiment of the present invention, the titanium is electropolished to obtain a TiO₂ surface of the uncoated dental implant.

In one embodiment of the present invention, a layer containing an adhesion promoter is provided between the surface of the uncoated dental implant and the biocompatible coating. The adhesion promoter, or spacer, is, for example, an organosilicon compound, preferably an amino-terminated silane or a compound based on an aminosilane, or an alkylphosphonic acid. Aminopropyl trimethoxysilane is especially preferred.

The adhesion promoter particularly improves the adhesion of the biocompatible coating to the surface of the dental implant material through coupling of the adhesion promoter to the surface of the dental implant material, by, for instance, ionic and/or covalent bonds, and through further coupling of the adhesion promoter to reactive components, particularly to the polymer of the biocompatible coating, by, for instance, ionic and/or covalent bonds.

The surprisingly improved take of the gingiva onto the dental implant of the present invention may be based on, but not limited, to a mechanism that the biocompatible coating of the dental implant according to the invention adsorbs reversible native proteins without denaturation, resulting in an imitataion of a biological and physiological surface. This unique property of the dental implant of the present invention allows an improved and accelerated take of the gingiva onto the dental implant without the deepening of the gingival sulcus. Moreover, bacterial infections of the gingiva can be prevented at the dental implant of the present invention upon its implantation into a patient, and the long-term tolerance of the dental implant of the present invention can be improved.

The present invention will now be further illustrated in the following examples, without being limited thereto.

### EXAMPLE 1

### Cell testings:

Coated and non-coated titanium plates were tested concerning their biocompatibility.

The test was performed with HEKn-Keratinozytes with a density of 30000 cells/cm² on different coated and non-coated titanium plates. The inkubation of the cells was performed in EpiLife-medium at 37°C at 5% C0₂ athmosphere in an incubator. The proliferation of new cells was measured by marking novell generated cells during the trialphase with Brom-desoxy-Uridin and comparing the intensity of the via antibody reaction generated colour in an Elisa-Reader at 620 nm.

### Results:

The Polyzene-F coated titanium plates showed after 24 h a significantly higher number of newly generated cells than found on the bare titanium. Ti = 100 % versus Ti-Polyzene-F = 150 %

### EXAMPLE 2

15 conventional dental implants (commercially available from Dr. Ihde Dental GmbH, Munich, Allfit RTM STI, Size 4.1 mm diameter, length 11 and 13 mm, respectively) made from pure titanium were electropolished (commercially available from Admedes Schüßler GmbH, Pforzheim). This procedure provides a pure TiO₂ surface. Highly purified linear Polyzene-F (bis-poly-trifluor-ethoxy-phosphazene, commercially available from Polyzenix GmbH, Ulm) having a molecular weight of more than 12 millions and a CI-concentration of below 0.0005%, was applied to the whole surface of the dental implants. 15 dental implants without any coating were used as controls. Each of the dental implants were implanted into the jawbone of patients and the tissue integration of the gingiva onto the dental implants was evaluated.

Approximately 8 weeks after implantation the dental implants according to the present invention showed a complete take of the gingiva onto the dental implants and no gingival sulcus were observed. In contrast thereto, the dental implants of the control group showed no sufficient take of the gingiva onto the dental implants and clear formations of gingival sulcus with a depth of 2 mm or more, in some cases already accompanied by bacterial infections, were observed in said patients.

The dental implants according to the present invention improve drastically the take of the gingiva onto the dental implants upon implantation into a patient. This surprising result prevents the deepening of the gingival sulcus, and thereby the risk of bacterial infections can be minimized, if not prevented. As a consequence, the sufficient take of the gingiva onto the dental implants of the present invention substantially reduces or prevents the loss of said dental implant and improves the long-term tolerance of said dental implant.

## Claims

1. A dental implant comprising:
a dental implant material comprising titanium; and
a biocompatible coating applied to the surface of the dental implant material that contacts the gingival of a patient upon implantation of the dental implant, wherein the biocompatible coating comprises a polymer of the general formula (I) 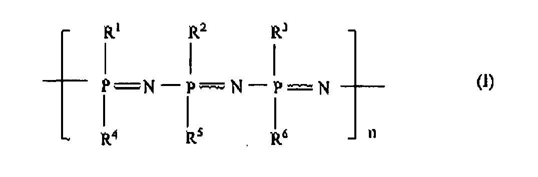 wherein n is from 2 to ∞,
the groups R¹ to R⁶ are the same or different, and represent an alkoxy, alkylsulfonyl, dialkylamino or aryloxy group, or a heterocycloalkyl or heteroaryl group with nitrogen as the heteroatom.

2. The dental implant of Claim 1, wherein at least one of the groups R¹ and R² is an alkoxy group substituted with at least one fluorine group.

3. The dental implant of Claim 1, wherein the alkoxy group is substituted with straight-chain or branched-chain alkyl groups having 1-20 carbon atoms, wherein the alkyl group is substituted with at least one halogen atom.

4. The dental implant of Claim 1, wherein the alkoxy group is selected from methoxy, ethoxy, propoxy, and butoxy groups, and wherein at least one alkoxy group is substituted with at least one fluorine atom.

5. The dental implant of Claim 1, wherein the aryl group in the aryloxy group is substituted with at least one alkoxy group selected from methoxy, ethoxy, propoxy, and butoxy groups, and wherein at least one alkoxy group is substituted with at least one fluorine atom.

6. The dental implant of Claim 1, wherein the polymer comprises a bis-poly-trifluorethoxy-polyphosphazene.

7. The dental implant of Claim 1, wherein the titanium is electropolished to obtain a TiO₂ surface of the uncoated dental implant.

8. The dental implant of Claim 1, wherein a layer containing an adhesion promoter is provided between the surface of the uncoated dental implant and the biocompatible coating.

9. The dental implant of Claim 1, wherein the biocompatible coating has a thickness from 1 nm to 100 µm.

10. The dental implant of Claim 1, wherein the biocompatible coating has a thickness from 10 nm to 10 µm.

11. The dental implant of Claim 1, wherein the biocompatible coating has a thickness up to 1µm.

12. The dental implant of Claim 1, wherein the biocompatible coating adsorbs reversible native proteins without denaturation, resulting in an imitation of a biological and physiological surface, and allows an improved and accelerated take of the gingiva onto the dental implant without the formation of gingival sulcus.

13. A method for manufacturing a dental implant comprising:
applying a biocompatible coating to a surface of a dental implant material that contacts the gingiva of a patient upon implantation of the dental implant,
wherein the dental implant material comprises titanium,
wherein the biocompatible coating comprises a polymer of the general formula (I): 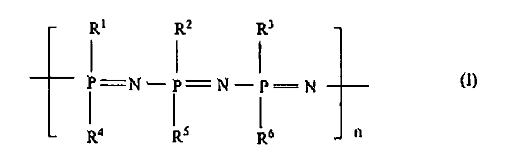 wherein n is from 2 to ∞,
the groups R¹ to R⁶ are the same or different, and represent an alkoxy, alkylsulfonyl, dialkylamino or aryloxy group, or a heterocycloalkyl or hetaroaryl group with nitrogen as the heteroatom.

## Patentansprüche

1. Dentalimplantat mit:
einem Dentalimplantat-Material, das Titan umfasst; und
einer biokompatiblen Beschichtung, die auf die Oberfläche des Dentalimplantat-Materials aufgebracht ist, die mit dem Zahnfleisch eines Patienten bei der Implantation des Dentalimplantats in Kontakt kommt, wobei die biokompatible Beschichtung ein Polymer der allgemeinen Formel (I) umfasst: 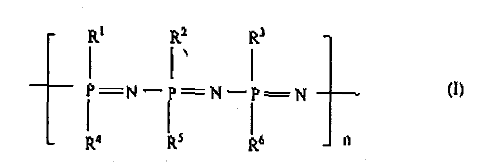 worin n von 2 bis ∞ ist,
die Gruppen R¹ bis R⁶ gleich oder verschieden sind und eine Alkoxy-, Alkylsulfonyl-, Dialkylamino- oder Aryloxy-Gruppe darstellen oder eine Heterocycloalkyl oder Heteroaryl-Gruppe mit Stickstoff als Heteroatom darstellen.

2. Dentalimplantat nach Anspruch 1, bei der zumindest eine der Gruppe R¹ und R² eine Alkoxy-Gruppe ist, die mit zumindest einer Fluor-Gruppe substituiert ist.

3. Dentalimplantat nach Anspruch 1, bei dem die Alkoxy-Gruppe durch geradkettige oder verzweigtkettige Alkyl-Gruppen mit 1 bis 20 Kohlenstoffatomen substituiert ist, wobei die Alkyl-Gruppe mit zumindest einem Halogen-Atom substituiert ist.

4. Dentalimplantat nach Anspruch 1, bei dem die Alkoxy-Gruppe aus Methoxy-, Ethoxy-, Propoxy- und Butoxy-Gruppen ausgewählt ist, und bei dem zumindest eine Alkoxy-Gruppe mit zumindest einem Fluor-Atom substituiert ist.

5. Dentalimplantat nach Anspruch 1, bei dem die Aryl-Gruppe in der Aryloxy-Gruppe durch zumindest eine Alkoxy-Gruppe substituiert ist, die aus Methoxy-, Ethoxy-, Propoxy- und Butoxy-Gruppen ausgewählt ist, und bei dem zumindest eine Alkoxy-Gruppe mit zumindest einem Fluor-Atom substituiert ist.

6. Dentalimplantat nach Anspruch 1, bei der das Polymer Bis-Polytrifluorethoxy-Polyphosphazen umfasst.

7. Dentalimplantat nach Anspruch 1, bei dem das Titan elektrolytisch poliert wird, um eine TiO₂-Oberfläche des unbeschichteten Dentalimplantats zu erzielen.

8. Dentalimplantat nach Anspruch 1, bei der ein Haftvermittler zwischen der Oberfläche des unbeschichteten Dentalimplantats und der biokompatiblen Beschichtung vorgesehen ist.

9. Dentalimplantat nach Anspruch 1, bei dem die biokompatible Beschichtung eine Dicke von 1 nm bis 100 µm aufweist.

10. Dentalimplantat nach Anspruch 1, bei dem die biokompatible Beschichtung eine Dicke von 10 nm bis 10 µm aufweist.

11. Dentalimplantat nach Anspruch 1, bei dem die biokompatible Beschichtung eine Dicke von bis zu 1 µm aufweist.

12. Dentalimplantat nach Anspruch 1, bei dem die biokompatible Beschichtung reversible native Proteine ohne Denaturierung adsorbiert, was zu einer Nachbildung einer biologischen und physiologischen Oberfläche führt und eine verbesserte und beschleunigte Aufnahme des Zahnfleisches auf dem Dentalimplantat ohne die Bildung von Zahnfleisch-Spalten ermöglicht.

13. Verfahren zur Herstellung eines Dentalimplantats mit den folgenden Schritten:
Aufbringen einer biokompatiblen Beschichtung auf eine Oberfläche eines Dentalimplantat-Materials, die mit dem Zahnfleisch eines Patienten nach der Implantation des Dentalimplantats in Kontakt kommt,
wobei das Dentalimplantat-Material Titan umfasst,
wobei die biokompatible Beschichtung ein Polymer der allgemeinen Formel (I) umfasst: 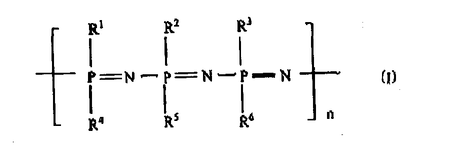 worin n von 2 bis ∞ ist,
die Gruppen R¹ bis R⁶ gleich oder verschieden sind und eine Alkoxy-, Alkylsulfonyl-, Dialkylamino- oder Aryloxy-Gruppe oder eine Heterocycloalkyl oder Hetaroaryl-Gruppe mit Stickstoff als dem Heteroatom darstellen.

## Revendications

1. Implant dentaire comprenant :
un matériau d'implant dentaire comprenant du titane, et
un revêtement biocompatible appliqué à la surface du matériau d'implant dentaire qui est en contact avec la gencive d'un patient lors de l'implantation de l'implant dentaire,
dans lequel le revêtement biocompatible comprend un polymère de formule générale (I) 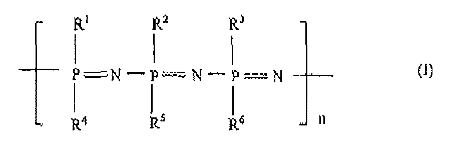 dans laquelle n vaut de 2 à ∞,
les groupes R¹ à R⁶ sont identiques ou différents, et représentent un groupe alcoxy, alkylsulfonyle, dialkylamino ou aryloxy, ou un groupe hétérocycloalkyle ou hétéroaryle ayant un azote à titre d'hétéroatome.

2. Implant dentaire selon la revendication 1, dans lequel au moins un des groupes R¹ et R² est un groupe alcoxy substitué par au moins un groupe fluor.

3. Implant dentaire selon la revendication 1, dans lequel le groupe alcoxy est substitué par des groupes alkyle à chaîne droite ou à chaîne ramifiée ayant de 1 à 20 atomes de carbone, dans lequel le groupe alkyle est substitué par au moins un atome d'halogène.

4. Implant dentaire selon la revendication 1, dans lequel le groupe alcoxy est choisi parmi les groupes méthoxy, éthoxy, propoxy et butoxy, et dans lequel au moins un groupe alcoxy est substitué par au moins un atome de fluor.

5. Implant dentaire selon la revendication 1, dans lequel le groupe aryle dans le groupe aryloxy est substitué par au moins un groupe alcoxy choisi parmi les groupes méthoxy, éthoxy, propoxy et butoxy, et dans lequel au moins un groupe alcoxy est substitué par au moins un atome de fluor.

6. Implant dentaire selon la revendication 1, dans lequel le polymère comprend un bis-polytrifluoroéthoxy-polyphosphazène.

7. Implant dentaire selon la revendication 1, dans lequel le titane est électropoli pour obtenir une surface d'implant dentaire non revêtu du type TiO₂.

8. Implant dentaire selon la revendication 1, dans lequel une couche contenant un accélérateur d'adhérence est intercalée entre la surface de l'implant dentaire non revêtu et le revêtement biocompatible.

9. Implant dentaire selon la revendication 1, dans lequel le revêtement biocompatible a une épaisseur de 1 nm à 100 µm.

10. Implant dentaire selon la revendication 1, dans lequel le revêtement biocompatible a une épaisseur de 10 nm à 10 µm.

11. Implant dentaire selon la revendication 1, dans lequel le revêtement biocompatible a une épaisseur maximale de 1 µm.

12. Implant dentaire selon la revendication 1, dans lequel le revêtement biocompatible adsorbe les protéines natives réversibles sans les dénaturer, avec pour résultat d'imiter une surface biologique et physiologique, et permet une prise améliorée et accélérée de la gencive sur l'implant dentaire sans formation de sillon gingival.

13. Procédé de fabrication d'un implant dentaire comprenant :
l'application d'un revêtement biocompatible à une surface d'un matériau d'implant dentaire qui est en contact avec la gencive d'un patient lors de l'implantation de l'implant dentaire,
dans lequel le matériau d'implant dentaire comprend du titane,
dans lequel le revêtement biocompatible comprend un polymère de formule générale (I) 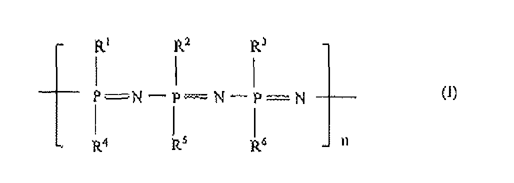 dans laquelle n vaut de 2 à ∞,
les groupes R¹ à R⁶ sont identiques ou différents, et représentent un groupe alcoxy, alkylsulfonyle, dialkylamino ou aryloxy, ou un groupe hétérocycloalkyle ou hétéroaryle ayant un azote à titre d'hétéroatome.
